# EUROPEAN PATENT SPECIFICATION

(11) **EP 2 514 429 B1**
(45) Date of publication and mention of the grant of the patent: **07.03.2018**
(21) Application number: 10825268.5
(22) Date of filing: 07.10.2010
(51) Int. Cl.: A61K 36/18, A61K 35/644, A61K 31/375, A61K 31/201, A61P 39/06, A61K 31/352, A61K 31/355

(54) **ANTIOXIDANT**
ANTIOXIDANS
AGENT ANTIOXYDANT

(30) Priority: 21.10.2009 RU 2009138990
(43) Date of publication of application: 24.10.2012
(73) Proprietor: Obshestvo S Ogranichennoj Otvetstvennostju "PARAFARM", Penza 440023 (RU)
(72) Inventor: ELISTRATOV, Dmitriy Gennadjevich, Penza 440060 (RU)
(74) Representative: Engel, Christoph Klaus
(86) International application number: PCT/RU2010/000563
(87) International publication number: WO 2011/049484

(56) References cited:
- WO-A1-2007/076534
- GB-A- 2 385 768
- RU-C2- 2 187 319
- SU-A3- 1 836 094
- US-A1- 2009 169 684
- DATABASE WPI Week 200842 Thomson Scientific, London, GB; AN 2008-G62384 XP002698627, & JP 2008 048729 A (MORIKAWA KENKODO KK) 6 March 2008 (2008-03-06)
- DATABASE WPI Week 200062 Thomson Scientific, London, GB; AN 2000-646096 XP002698628, & RU 2 149 566 C1 (KONDRATEVA I I) 27 May 2000 (2000-05-27)
- DATABASE WPI Week 200826 Thomson Scientific, London, GB; AN 2008-D64088 XP002698629, & RU 2 315 593 C1 (ALMETEVSKY NASOSNY WKS STOCK CO) 27 January 2008 (2008-01-27)
- HARAGUCHI H ET AL: "Protection against oxidative damage by dihydroflavonols in Engelhardtia chrysolepis.", BIOSCIENCE, BIOTECHNOLOGY, AND BIOCHEMISTRY JUN 1996, vol. 60, no. 6, June 1996 (1996-06), pages 945-948, XP002698630, ISSN: 0916-8451
- LEBLANC B W ET AL: "Antioxidant activity of Sonoran Desert bee pollen", FOOD CHEMISTRY, ELSEVIER LTD, NL, vol. 115, no. 4, 15 August 2009 (2009-08-15), pages 1299-1305, XP026077373, ISSN: 0308-8146, DOI: 10.1016/J.FOODCHEM.2009.01.055 [retrieved on 2009-01-27]
- 'Vse o mede i produktakh pchelovodstva.' PYLTSA, MATOCHNOE MOLOCHKO, 2005-2008 G, [Online] Retrieved from the Internet: <URL:http://honey-online.narod ru//pollen_and_perga html>

## Description

The invention relates to the pharmaceutical industry for producing medicaments with antioxidant action.

One of the main causes of ageing is the intoxication of human organs from the products of vital activity and inability of the human excretory system to purify (excrete) poisons and toxins. (Academician Amosov N.M., Coping with Age, Moscow, 1996, Bud Zdorov Publishers). One of the causes of disorders in the excretory system is the deficiency of nutrients essential for a human so that its organs are short of essential substances while the body receives substances which are already in excess. As a result, the body becomes "slagged". Therefore, it is a prime object of the medical science to regulate the functions of the excretory system using microdoses of essential substances which upon entry in the body contribute to normalization of the excretory system functions leading to the body purification from slagging and toxins.

Known in the prior art is the Elton natural polyvitaminic complex (RU 2062585, IPC A23L 1/076, 1/302) comprising pollen, eleutherococcus, ascorbic acid (vitamin C), α-tocopherol acetate (vitamin E), propolis, calcium stearate, talc and lactose. Pollen is regarded as an energy source in Elton (pollen is a natural concentrate of bioactive compounds, proteins, macro- and micronutrient elements including iodine, selenium, zinc, ferrum and others. It contains 20 nonessential amino acids and essential amino acids, 28 micronutrient elements, provitamin A, vitamins B, D, P, PP, K, flavonoids, phytoncids, enzymes) and Elton is a combined adaptogene immunomodulator eleutherococcus containing strictly dosed vitamins.

The prior art closest to the invention by its technical essence and the achieved effect is SU 1836094 A3, 23.08.1993 disclosing an immunostimulant with antioxidant action and comprising propolis, pollen, royal jelly, dry eleutherococcus or ginseng extract, dry Leuzea or Rhodiolae rosea extract, honey.

However, the above drug is contraindicated for patients suffering from cardiovascular diseases, especially elderly people since it contains eleutherococcus which may lead to an increase in the arterial tension.

It is an object of the present invention to diversify the range of antioxidants of natural origin for enabling a better immunity and protecting against free radicals.

The above object is achieved as follows.

An antioxidant is provided comprising pollen, royal jelly, vitamin C, vitamin E, dihydroquercetin and excipients at the following ratio (wt. %):
pollen: 5-50
dihydroquercetin: 5-50
vitamin C: 5-10
vitamin E: 0.05-5
royal jelly: 0.05-5
excipients: balance.

The antioxidant may be shaped as tablets.

Dihydroquercetin, a bioflavonoid derived from Siberian larch, is a reference antioxidant. It has powerful anti-inflammatory and antiallergic properties, strengthens and recovers connective tissues, contributes to lowering the level of cholesterol, potentiates many valuable substances (vitamin C and vitamin E); strengthens blood vessels and capillaries, improves blood microcirculation, prevents thrombosis, reduces inflammatory events in prostate, strengthens immunity, protects stomach and liver against adverse effects, activates regeneration processes of stomach mucosa, provides manifested prophylaxis of major age-related diseases such as cancer, cardiovascular diseases, diseases of the brain and others, improves the resistance of body tissues to a damaging action of the excess blood sugar, lowers the risk of diabetes and alleviates the course of already developed forms thereof, positively acts on the nervous system, activates neural processes.

As a result of the search carried out in the sources of scientific-technical and patent literature, no composition has been found having a similar combination of essential features whereby the same positive effect is achieved. Therefore, the proposed invention offers a technical solution which is novel, has inventive step and is industrially applicable.

The antioxidant is available as tablets. Substance essential for this dosage form are use as excipients. The lower quantitative limit is defined by the ease of use of the proposed complex, i.e., application of a less number of tablets. The upper quantitative limit is defined by the difficulty of shaping a tablet at a higher percentage ratio because it would fall to pieces.

The invention will be further illustrated by the following examples.

### Example 1

An exemplary formulation of the complex for a 500 mg tablet:
pollen: 100 mg
dihydroquercetin: 30 mg
vitamin C: 30 mg
vitamin E: 5 mg
royal jelly: 1 mg
excipients: 334 mg.

### Example 2

An exemplary formulation of the complex for a 500 mg tablet:
pollen: 200 mg
dihydroquercetin: 50 mg
vitamin C: 30 mg
vitamin E: 5 mg
royal jelly: 1 mg
excipients: 214 mg.

The medicament has been tested in elderly volunteers, mainly in the group of hypertensive patients.

As a result of the conducted treatment, it may be concluded that said medicament is indicated for elderly people, namely, the inventive formulation both qualitatively and quantitatively prevents the mains causes of ageing.

The proposed formulation of the medicament supplies the body with microdoses of all essential substances and provides antioxidative protection against external adverse factors.

## Claims

1. An antioxidant compound for use in protecting the human body against free radicals, comprising pollen, and royal jelly, **characterized in that** it further includes vitamin C, vitamin E, dihydroquercetin and excipients at the following ratio (wt. %):
pollen: 5-50
dihydroquercetin: 5-50
vitamin C: 5-10
vitamin E: 0.05-5
royal jelly: 0,05-5
excipients: balance.

2. The antioxidant compound for use according to claim 1, **characterized in that** it is available as a tablet.

## Patentansprüche

1. Antioxidationsmittel zur Verwendung für den Schutz des menschlichen Körpers vor freien Radikalen, wobei das Antioxidationsmittel Pollen und Gelee Royale umfasst, **dadurch gekennzeichnet, dass** es weiterhin Vitamin C, Vitamin E, Dihydroquercetin und Hilfsstoffe in folgendem Verhältnis umfasst (Gewichtsprozent):
Pollen: 5-50
Dihydroquercetin: 5-50
Vitamin C: 5-10
Vitamin E: 0,05-5
Gelee Royale: 0,05-5
Hilfsstoffe: restliche Anteile.

2. Antioxidationsmittel nach Anspruch 1, **dadurch gekennzeichnet, dass** es als eine Tablette ausgebildet ist.

## Revendications

1. Composé antioxydant destiné à être utilisé dans la protection du corps humain contre les radicaux libres, comprenant du pollen et de la gelée royale, **caractérisé en ce qu'**il comprend en outre de la vitamine C, de la vitamine E, de la dihydroquercétine et des excipients au rapport suivant (% en poids) :
pollen : 5 - 50
dihydroquercétine : 5 - 50
vitamine C : 5 - 10
vitamine E : 0,05 - 5
gelée royale : 0,05 - 5
excipients : reste.

2. Composé antioxydant destiné à être utilisé selon la revendication 1, **caractérisé en ce qu'**il est disponible sous forme de comprimé.
